# EUROPÄISCHE PATENTANMELDUNG

(11) **EP 3 069 666 A1**
(43) Veröffentlichungstag der Anmeldung: **21.09.2016**
(21) Anmeldenummer: 15000789.6
(22) Anmeldetag: 17.03.2015
(51) Int. Cl.: A61B 17/17

(54) **VERFAHREN UND VORRICHTUNG ZUR WIEDERHERSTELLUNG DES KREUZBANDES ACL IM KNIE**

(71) Anmelder: Lei-Schruff, Lian, 86529 Schrobenhausen (DE)
(72) Erfinder: Lei, Jun Hu, Chengdu City (CN); Hu, Yong, 610041 Chengdu (CN)

(57) **Zusammenfassung**

Die Erfindung bezieht sich auf ein Verfahren und eine Vorrichtung zur Wiederherstellung eines gerissenen Kreuzbandes ACL im menschlichen Knie.

## Beschreibung

Die Erfindung bezieht sich auf ein Verfahren und eine Vorrichtung zur Wiederherstellung eines gerissenen Kreuzbandes ACL im menschlichen Knie.

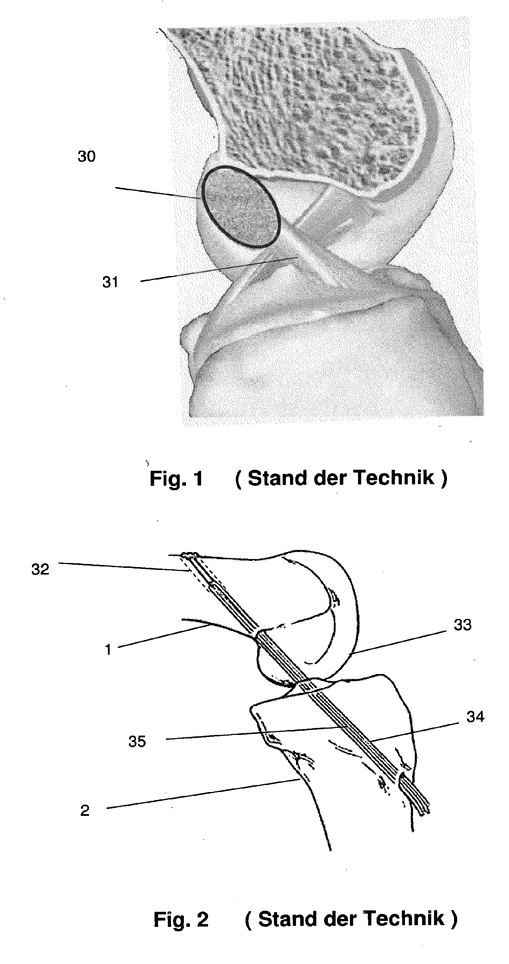

Nach dem Stand der Technik wird bei gerissenen Kreuzbändern im Kniebereich die nachfolgend beschriebene Operationsmethode durchgeführt. Zur besseren Erläuterung dienen die Fig. 1 und 2, die den Stand der Operationstechnik wiedergeben.

Fig. 1 zeigt ein Kniegelenk mit ACL- und PCL-Kreuzbändern. Von größerer Bedeutung ist die Wiederherstellung des ACL-Kreuzbandes.

Beim der wie in Fig. 2 dargestellten Operationstechnik wird seitlich am Oberschenkelknochen 1 eine Bohrung 32 angesetzt, die durch den Knochen geführt wird und zwischen den beiden Knochenwangen 33 aus dem Oberschenkelknochen austritt.

Ebenso wird seitlich vom Schienbein 2 eine Bohrung 34 angesetzt, die einen Austrittspunkt in der Kontaktfläche zwischen Oberschenkelknochen und Schienbeinknochen hat.

Durch diese beiden Bohrungen, deren Austrittsöffnungen passend zueinander angeordnet sind, werden die Ersatzkreuzbänder 35 eingezogen. Nachdem sie in der Seitenwand des Oberschenkelknochens verankert sind, werden sie angespannt.

Der Durchmesser dieser Bohrungen 32 und 34 liegt bei ca. 8 bis 9 mm. Als Ersatzkreuzbänder können künstliche oder körpereigene Gewebe des Patienten verwendet werden.

Bei der Ausführung nach dem Stand der Technik zeigen sich einige Nachteile.

So ist der Erfolg der Methode ist stark abhängig vom handwerklichen Geschick des Operateurs.

Beim Austritt der im Durchmesser verhältnismäßig großen Bohrung 32 in der Kortikalis der Kontaktfläche zwischen den beiden Knochen können sehr leicht die Ränder der Bohrung im Umfangsbereich ausbrechen. In der dünnen Schicht der Kortikalis entstehen dabei oft keine glatten Bohrungen, sondern raue und unregelmäßig geformte Bohrlochwandungen.

Das kann dazu führen, dass ein erhöhter Verschleiß an den neu eingesetzten Kreuzbänder auftritt.

Eine weitere Schwierigkeit ergibt sich beim Spannen der Bündel 35, die im wesentlichen einen kreisförmigen Querschnitt haben. Diese können sich bald nach der Operation lockern und für die relativ großen Bündeldurchmesser zeigt sich erhöhter Verschleiß in den Umlenkungselementen der Endverankerungen an den Seitenwänden der Knochen.

In Fig. 1 ist gezeigt, dass die bandförmigen Kreuzbänder in einem elliptischen Querschnitt 30 am Knochen angewachsen sind. Der bandförmige Querschnitt der natürlich angewachsenen Kreuzbänder bietet deutlich höhere Stabilität für das Kniegelenk als der runde Querschnitt der Ersatzkreuzbänder.

Die Aufgabe der Erfindung liegt darin, einen der Natur besser angepassten Einbau der Kreuzbänder zu bewirken, mit dem Ziel, mehr Stabilität und weniger Verschleiß nach der Operation zu erhalten.

Die Lösung der Aufgabe erfolgt entsprechend den Merkmalen für die Verfahrens- und Vorrichtungsansprüche und wird anhand der Figuren 3 bis 13 erläutert.
Fig. 3 zeigt eine transparente Durchsicht durch einen Oberschenkelknochen von unten, mit Blick auf die Kontaktfläche des Oberschenkels mit der erfindungsgemäßen Anordnung der Bohrungen und deren Austrittsöffnungen.
Fig. 4 zeigt den Fußspurbereich und die Anordnung, wie die erfindungsgemäßen Bohrungen im Kontaktbereich des Oberschenkels aus der Kortikalis austreten.
Fig. 5 zeigt ein erfindungsgemäßes Ausführungsbeispiel der Vorrichtung zur bevorzugten Anordnung der Bohrungen am Oberschenkelknochen.
Fig. 6 zeigt eine 3-D-Darstellung der erfindungsgemäßen Anwendung und Vorrichtung am Oberschenkel.
Fig. 7 zeigt ein erfindungsgemäßes Ausführungsbeispiel für die Anwendung am Schienbein.
Fig. 8 und 9 zeigen eine zugehörige 3-D-Darstellung.
Fig. 10 zeigt die erfindungsgemäßen Bohrungen, wie sie im Schienbein angeordnet werden.
Fig. 11 zeigt eine bevorzugte Anordnung der Austrittsbohrungen in der Kontaktfläche des Schienbeins.
Fig. 12 zeigt eine erfindungsgemäße Vorrichtung zur Herstellung der erfindungsgemäßen Schrägbohrungen.
Fig. 13 zeigt eine Vorrichtung zur Herstellung einer dünnen Führungsbohrung.

Die Besonderheit der Erfindung besteht darin, dass beim Einbau des Ersatzkreuzbandes im menschlichen Knie vom Seitenbereich des Oberschenkelknochens 1 und/oder vom Seitenbereich des Schienbeinknochens 2 eine Ansatzbohrung 10, 11 mit einem größeren Durchmesser 4, 5 eingebracht wird, die bevorzugterweise in der Spongiosa endet und nicht durch die Kortikalis und die Knochenhaut austritt und dass durch diese Ansatzbohrungen 10, 11 bezüglich deren Bohrachse mehrere Schrägbohrungen 6, 7 eingebaut werden, die einen kleineren Durchmesser haben als die Ansatzbohrungen 10, 11 und dass diese kleineren Bohrungen 6, 7 so nach außen geneigt sind, dass ihre Austrittsöffnungen 20, 21, 22, 23, 40, 41, 42, 43 in der Kortikalis und Knochenhaut auf einer bevorzugten ovalen Fläche 24, 44 liegen, die größer ist als die Querschnittsfläche der Ansatzbohrungen 10,11.

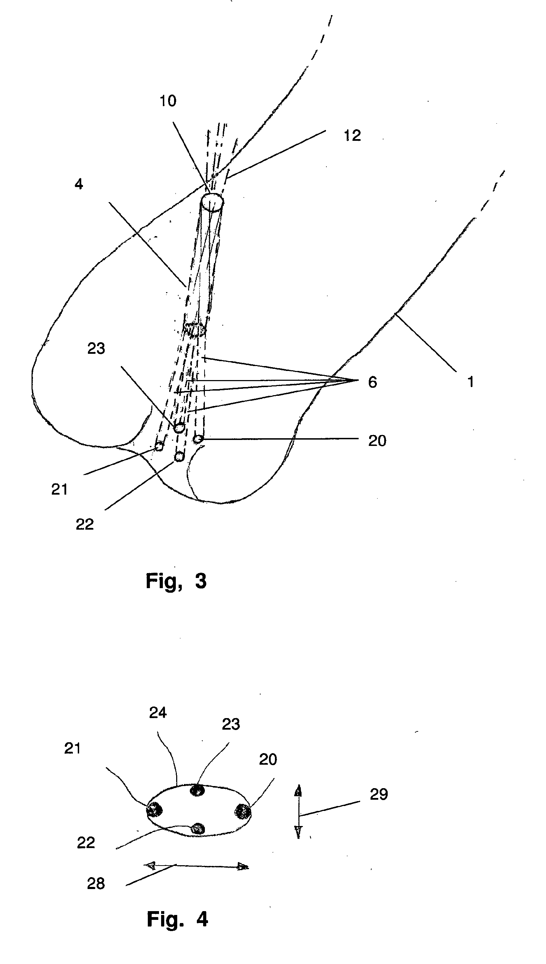

Dieser Sachverhalt wird in Fig. 3 für den Oberschenkelknochen dargestellt.

Das Besondere bei dieser erfindungsgemäßen Ausführung liegt darin, dass von der Seitenfläche des Knochens aus zuerst eine relativ große Ansatzbohrung 10 durch die Kortikalis ins Innere der Spongiosa des Oberschenkelknochens eingebohrt wird. Die Bohrtiefe ist jedoch so kurz gewählt, dass diese große Ansatzbohrung 10 nicht die Kortikalis durchdringt, die sich im Bereich der gegenüberliegenden Austrittsfläche oder Kontaktfläche des Knochens befindet. Die Bohrung endet einige mm vorher im Übergangsbereich zwischen Spongiosa und Kortikalis. So entsteht keine große Öffnung in der Kontaktfläche.

Nach Entfernung der Spongiosa aus der Ansatzbohrung 10 , wird mit mehreren kleineren, schrägen Bohrungen 6 weitergearbeitet, die schliesslich die Kortikalis in der Kontaktfläche durchdringen.

Der Bohrdurchmesser 4 der größeren Ansatzbohrungen 10 liegt in einem Bereich zwischen 8 und 10 mm.

Die kleineren Schrägbohrungen 6 werden bevorzugterweise mit Durchmessern zwischen 2 und 5 mm ausgeführt.

Das Besondere ist, dass diese kleinen Bohrungen 6 schräg nach außen geführt werden. Die Bohrachsen 12 dieser schrägen kleinen Bohrungen 6 sind nach außen gerichtet. Mit diesen kleinen Schrägbohrungen 6 wird zunächst die Spongiosa und anschließend die Kortikalis im Bereich der Gelenkkontaktfläche des Oberschenkelknochens durchbohrt. Diese kleineren Bohrungen werden beispielsweise mit einer Kirschner-Nadel oder mit vergleichbaren Bohrwerkzeugen kleinen Durchmessers nach dem Stand der Technik hergestellt.

Um der natürlichen Befestigung der Kreuzbänder möglichst nahe zukommen, wird die Neigung 12 der einzelnen kleinen Bohrungen 6 so gewählt, dass die Austrittsöffnungen 20, 21, 22, 23 in der Kortikalis der Kontaktfläche des Oberschenkelknochens möglichst auf einer ellipsenartigen Fläche 24 bzw. auf deren Umfanglinie liegen.

Fig. 4 zeigt eine derartige elliptische Fläche 24, deren Ellipsenachse 29 in einem bevorzugten Bereich zwischen 8 bis 10 mm liegt und deren größere Ellipsenachse 28 in einem bevorzugten Bereich zwischen 14 bis 20 mm liegt.

Besonders angestrebte Abmessungen für die Abstände gegenüberliegender Austrittsöffnungen 20, 21 liegen bei 9 mm, für die Austrittsöffnungen 22, 23 bei 16 mm.

Die angestrebte Lage der Austrittsöffnungen 23 und 22 wird mit der Uhrstellungen 12 Uhr und 6 Uhr verglichen, die Austrittsöffnungen in der größeren Ellipsenachse mit den Uhrstellungen 3 Uhr und 9 Uhr bezeichnet.

Dadurch, dass die in dem zunächst größeren Bohrlochdurchmesser 4 eingeführten Kreuzbandbündel auf mehrere kleine Bündel verteilt werden, wird eine höhere Stabilität, Flexibilität und Beweglichkeit im Kniegelenk erreicht, da diese Kreuzbänder über eine größere Fläche verteilt befestigt werden und die Kräfte besser verteilen können.

Des weiteren ist erfahrungsgemäß durch die Wahl kleinerer Bohrer mit Durchmessern von 2 bis 3 mm, wie beispielsweise mit Kirschner-Nadeln, die Gefahr weitaus geringer, dass unregelmäßige Bohrlochwandungen und Ausbrüche beim Durchdringen der Kortikalis auftreten.

Auf diese Weise wird der Verschleiß der einzelnen kleineren Kreuzbandbündel reduziert.

Ein weiterer Vorteil der Erfindung liegt darin, dass die auf mehrere Stränge aufgeteilten Kreuzbänder leichter und gleichmäßiger gespannt werden können. Die Gefahr, dass die Kreuzbänder sich vorzeitig lockern, wird deutlich reduziert.

Passend zu dem Fußspurbereich 30 des Oberschenkelknochens 1 werden nun kleine Bohrungen 7 im Schienbein 2 ausgeführt.

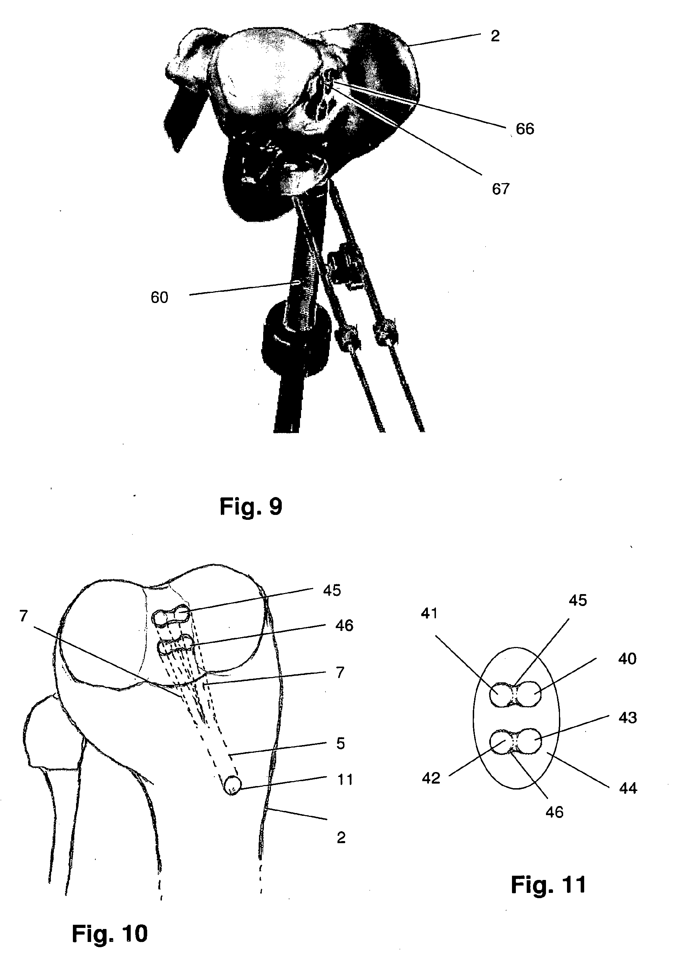

Fig. 10 und Fig. 11 zeigen Schienbeinknochen 2, bei denen mehrere erfindungsgemäße kleine Bohrungen 7 ausgeführt sind.

Zunächst wird eine größere Ansatzbohrung 11 in der Kortikalis des seitlichen Schienbeinknochens eingebracht.

Der Querschnitt 5 dieser größeren Ansatzbohrung 11 hat einen bevorzugten Durchmesser von 8 bis 10 mm. Diese Ansatzbohrung 11 wird nur so tief in die Spongiosa eingebracht, dass sie nicht aus der Kortikalis der Kontaktfläche zum Oberschenkelknochen austritt. Anschließend werden durch diese größere Ansatzbohrung 11 kleinere nach außen geneigte Bohrungen 7 ausgeführt, die in Austrittsöfffnungen 40, 41, 42, 43 durch die Kortikalis in der Kontaktfläche des Schienbeins austreten.

Auch diese Austrittsöffnungen liegen bevorzugterweise auf einer elliptischen Fläche 44 . Dabei ist die Spreizung dieser Austrittsöffnungen so gewählt, dass sie einen deutlich größeren Querschnitt bestreichen, als den Kreisquerschnitt 5 der großen Ansatzbohrung 11.

In einer bevorzugten Ausführungsvariante sind benachbarte Austrittsöffnungen 41, 40 und 42, 44 zu gemeinsamen Langlöchern 45 und 46 ausgebildet. Die Anordnung und Abstände dieser Langlöcher zueinander werden so gewählt, dass sie zu den Austrittsöffnungen 20, 21, 22, 23 des Oberschenkelknochens passend stehen.

Eine bevorzugte Abmessung der Langlöcher 45 und 46 liegt bei 7 x 3,5 mm.

Die Herstellung dieser Langlöcher 45 und 46 erfolgt dadurch, dass der Grat zwischen den entsprechenden Bohrungen mit besonderen Werkzeugen nach dem Stand der Technik herausgeschliffen wird. Dieses Herausschleifen kann auch dadurch geschehen, dass die Neigung eines Bohrers in der Öffnung 40 in Richtung der Bohrung 41 verschwenkt und dabei dieser Grat herausgeschliffen wird.

Auf diese Weise erhalten die Bohrlochwandungen der Langlöcher eine glatte Oberfläche, die verhindert, dass die Kreuzbandbündel einem größeren Verschleiß unterliegen.

Die kleineren Bohrungen 7 werden bevorzugterweise mit Kirschner-Nadeln hergestellt, die Bohrdurchmesser zwischen 2 und 4 mm aufweisen.

Wenn die kleineren Bohrungen 7 und 6 im Oberschenkel und Schienbein fertiggestellt sind, werden die Ersatzkreuzbänder eingezogen, verankert und gespannt.

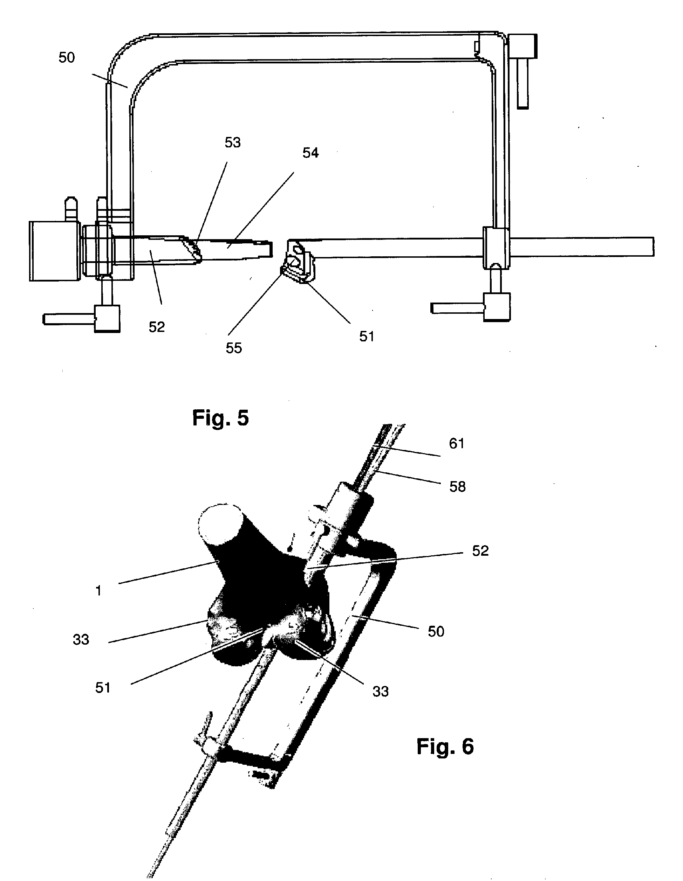

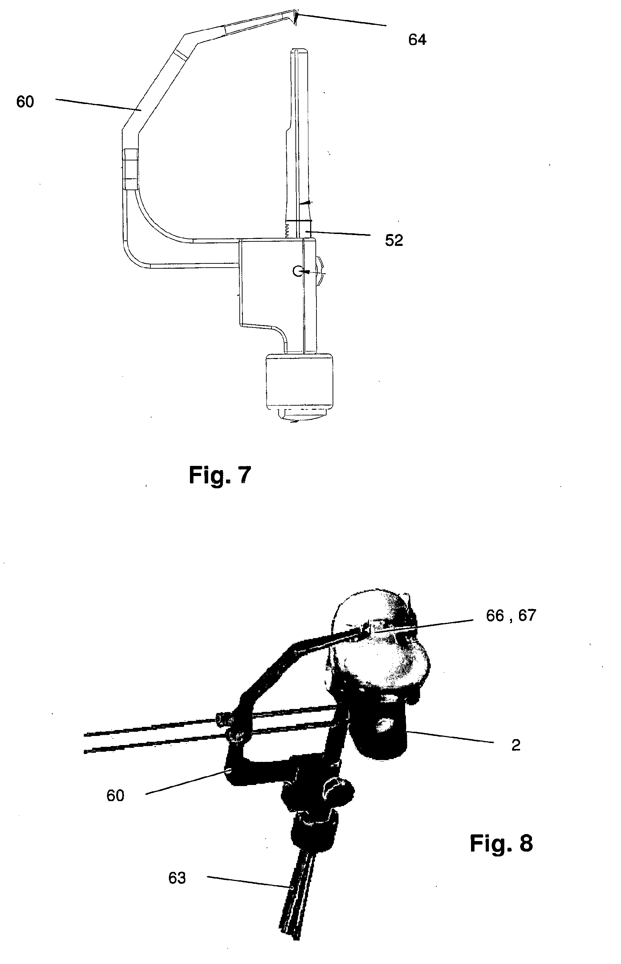

Die für das erfindungsgemäße Verfahren notwendigen Vorrichtungen sind für den Oberschenkelknochen in den Fig. 5, 6 und für das Schienbein in den Fig. 7, 8 und 9 dargestellt.

Die erfindungsgemäße Vorrichtung für das Verfahren besteht darin, dass sich an einem schraubenzwingenartigen Führungsrahmen 50 oder Bogenführungsrahmen 60 ein Arbeitsfutterrohr 52 mit Sägezähnen 53 befindet und dass auf der gegenüberliegenden Seite ein Festhalter 51 und/oder mindestens ein Spitzzahnhaken 64 angeordnet ist, die jeweils auf die Oberfläche der Knochen angepasst sind.

Am Oberschenkelknochen 1 wird der Führungsrahmen 50 befestigt. An diesem Führungsrahmen ist ein Arbeitsfutterrohr 52 befestigt, durch welches die Bohrungen ausgeführt werden. Am Ende dieses Arbeitsfutterrohres 52 befinden sich Sägezähne 53, die in die seitliche Kortikalis eingedrückt werden können, um dem Führungsrahmen 50 Halt zu geben.

Auf der gegenüberliegenden Seite des schraubzwingenartigen Führungsrahmens 50 befindet sich ein Festhalter 51. Dieser ist so ausgebildet, dass er zwischen die beiden Knochenwangen 33 des Oberschenkelknochens eingeführt werden kann. Zur leichteren Fixierung ist dieser Festhalter 51 mit schneidenartigen Elementen versehen, die ein Verrutschen des Festhalters 51 an der Knochenoberfläche verhindern.

Zudem befinden sich am Festhalter 51 Öffnungen 55, die ermöglichen, dass beim Einbringen der kleinen schrägen Bohrungen 6 die Bohrköpfe der Bohrer durch die Kortikalis der Kontaktfläche des Oberschenkelknochens 1 austreten können.

Um Schrägbohrungen 6, 7, mit kleinem Durchmesser im Arbeitsfutterrohr 52 zielgerecht ausführen zu können, wird nach der Herstellung der größeren Bohrung 10 und nach entfernen der Spongiosa in das Arbeitsfutterrohr 52 ein Führungselement 54 eingeführt.

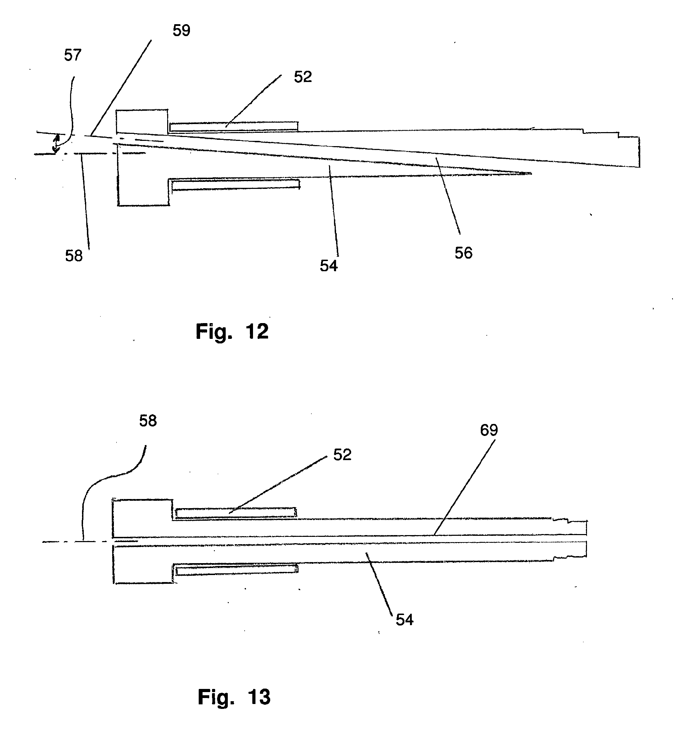

Ein solches Führungselement 54 ist in Fig. 12 dargestellt. Bei dem Führungselement 54 handelt es sich um eine längliche Vorrichtung, die geneigte Bohrungen oder Bohrführungen 56 für Bohrer 66, 67, mit kleinen Bohrdurchmessern enthält.

Das Führungselement 54 ist passgenau für den Innendurchmesser des Arbeitsfutterrohrs 52 gefertigt und kann darin gedreht und fixiert werden.

Die Bohrachsen 59 dieser Bohrführungen 56 sind unter einem Winkel 57 zur Achse des Arbeitsfutterrohrs 52 geneigt. Dieser Neigungswinkel 57 bewegt sich in einem Bereich von wenigen Grad und ist so gewählt, dass die kleinen Bohrungen 6,7, nach außen geneigt werden können, wenn sie die größere Bohrung 4, 5, verlassen.

Die kleineren Bohrungen 6 werden bevorzugterweise mit Kirschner-Nadeln hergestellt. Die Führung dieser Nadeln in dem länglichen Führungselement 54 erfolgt durch schräg angeordnete Bohrungen oder Längsnuten oder Führungsrillen 56 nach dem Stand der Technik.

Es kann sich dabei um kreisförmige Querschnitte oder teilkreisförmige, rillenartige Führungskanäle handeln.

Um die gewünschten Austrittsöffnungen nach Lage der Uhrzeit 12, 6, 3 und 9 Uhr einzustellen, wird das Führungselement 54 im Arbeitsfutterrohr 52 um dessen Längesachse gedreht. Auf diese Weise können die Austrittsöffnungen in der Kortikalis auf einer etwa ellipsenartigen Umfangslinie verteilt werden, wobei dann der Einbau der Ersatzbänder den natürlich angewachsenen Kreuzbändern nahe kommt und eine höhere Stabilität, Flexibilität und Beweglichkeit erreicht wird.

Um die Öffnungen der größeren Achse der Ellipse zu erreichen, ist es zweckmäßig, unterschiedliche Führungselemente 54 zu verwenden, in denen unterschiedlich geneigte Führungsbohrungen 56 angeordnet sind. Auch können Führungselemente 54 gleichzeitig mehrere Führungsbohrungen oder Rillen 56 enthalten, so dass mehrere kleine Bohrungen gleichzeitig ausgeführt werden können.

Der Ablauf bei der Wiederherstellung des gerissenen Kreuzbandes verläuft beispielhaft in folgenden Arbeitsschritten:
In einem ersten Arbeitsgang wird am Oberschenkel 1 ein Führungsrahmen 50 befestigt. Dieser schraubzwingenartige Führungsrahmen 50 besitzt auf der einen Seite ein Arbeitsfutterrohr 52 und auf seiner gegenüberliegenden Seite einen Festhalter 51. Zur Fixierung wird das Arbeitsfutterrohr 52, das auf einer Seite Sägezähne 53 besitzt, durch Zusammenspannen des Führungsrahmens 50 in die Kortikalis auf der Seitenfläche des Oberschenkelknochens eingedrückt. Die Fixierung auf der anderen Seite des Führungsrahmens 50 erfolgt dadurch, dass der Festhalter 51 zwischen die Wangen 33 des Oberschenkelknochens 1 eingedrückt wird. Die Form des Festhalters 51 ist dabei mit einer Oberfläche ausgestaltet, die ermöglicht, dass der Festhalter 51 rutschfest auf der Oberfläche des Oberschenkelknochens 1 aufliegt.

Nachdem der Führungsrahmen 50 so aus gerichtet ist, dass die später hergestellten kleinen Bohrungen in der richtigen Lage aus der Kontaktfläche zwischen den beiden Kniegelenkknochen austreten, wird in das Arbeitsfutterrohr 52 in einem ersten Arbeitsgang eine Zentriervorrichtung eingefügt, wie sie in Fig. 13 dargestellt ist. Dieses Führungselement 54 enthält eine zentrische Bohrung 69, die einen kleinen Bohrer oder eine Kirschner-Nadel mit einem bevorzugten Durchmesser zwischen 2 und 3 mm zentrisch führen kann.

Dieser kleine Bohrer oder diese Kirschner-Nadel wird zentrisch zum Arbeitsfutterrohr 52 durch die seitlich am Oberschenkelknochen liegende Kortikalis eingebohrt und dann durch die Spongiosa in Richtung der Kontaktfläche im Kniegelenk gebohrt, ohne dass jedoch die Kortikalis durchbohrt wird.

Diese erste kleine Bohrung dient als Zentrierbohrung. Entlang dieser kleinen Zentrier- oder Führungsbohrung wird anschließend eine Bohrung mit größerem Durchmesser 4 von der Seite des Oberschenkelknochens 1 zuerst durch die Kortikalis und anschließend durch die Spongiosa geführt. Auch diese Bohrung 10 mit größerem Durchmesser 4 verbleibt ebenso im Bereich der Spongiosa, ohne die gegenüberliegende Kortikalis zu durchbohren.

Damit diese Bohrung möglichst zielgenau wird, empfiehlt sich die Führung entlang der kleinen Bohrung, die durch eine Kirschner-Nadel erzeugt wurde.

Es kann zweckmäßig sein, dass diese Führungsnadel mit einem Hohlbohrer überbohrt wird. Der Durchmesser 4 dieser größeren Bohrung 10 liegt bevorzugterweise in einer Größenordnung zwischen 8 und 10 mm.

Nach Herstellung dieser größeren Bohrung 10 wird der Innenraum dieser Bohrung von der Spongiosa befreit.

In das Arbeitsfutterrohr 52 wird ein Führungselement 54 eingeführt, das schräge Führungsbohrungen oder Schlitze oder Rillen besitzt, die unter einem Winkel 57 zur Symmetrieachse des Arbeitsfutterrohres 52 geneigt sind. Mit Hilfe dieses Führungselementes 54 ist es möglich, dass kleinere Bohrungen 6 schräg nach außen gerichtet durch die Bohrung 10 weitergeführt werden können.

Diese kleineren Bohrungen 6 treten bevorzugterweise in vier voneinander beabstandeten Öffnungen 20, 21, 22, 23 aus der Kortikalis im Bereich der Kontaktfläche zwischen den beiden Knieknochen aus. Die Beabstandung der Austrittsöffnungen 20, 21, 22, 23 wird dadurch erreicht, dass zum einen die Führungselemente 54 um 180° gedreht werden, um die sich gegenüberliegenden Öffnungen zu erzeugen.

Ebenso können Führungselemente 54 mit verschieden geneigten Führungen 56 mit unterschiedlichen Winkeln 57 verwendet werden.

Diese auf einer Ellipse liegende Anordnung der aufgesplitteten Ersatzkreuzbänder kommt damit in die Nähe der Abmessungen, wie sie bei natürlich angewachsenen Kreuzbändern gegeben ist. Die Verteilung der Kräfte aus den einzelnen Kreuzbandbündeln führt zu seiner höheren Stabilität, Flexibilität und Beweglichkeit im Kniegelenk.

Dadurch, dass die Durchdringung der Kortikalis nur mit kleinen Bohrungen 6 erfolgt, die einen bevorzugten Bohrdurchmesser zwischen 2 und 4 mm aufweisen, lässt sich die Durchbohrung mit glatten Bohrlochrändern erreichen. Dies sorgt dafür, dass weniger Verschleiß an den Ersatzkreuzbändern auftritt.

Gegenüberliegende Austrittsöffnungen können in der Regel dadurch hergestellt werden, dass das Führungselement 54 im Arbeitsfutterrohr 52 um 180° gedreht wird. Je nachdem, wie groß der Abstand der Austrittsöffnungen 20, 21 bzw. 22, 23 ist, werden unterschiedliche Führungselemente 54 verwendet, bei denen die Neigung 12 der Bohrungen 6 unterschiedliche Winkel 57 aufweisen.

Am Schienbein wird das Verfahren bevorzugterweise in einer etwas modifizierten Weise durchgeführt.

Am Bogenführungsrahmen 60 wird ein Arbeitsfutterrohr 52 mit Sägezähnen 53 angeordnet, aber auf der gegenüberliegenden Seite des Führungsrahmens befinden sich ein oder mehrere Spitzzahnhaken 64. Diese sind auf der Oberfläche der Kontaktfläche des Schienbeinknochens besser zu verankern als die Festhalter 51.

In einem ersten Arbeitsgang wird der Bogenführungsrahmen 60 mit seinem Arbeitsfutterrohr 52, das wiederum Sägezähne besitzt, auf der Seitenfläche des Schienbeinknochens angesetzt und auf der gegenüberliegenden Seite mit dem Spitzzahnhaken an der Kontaktfläche des Schienbeinknochens verankert.

Nach zielgerechter Ausrichtung, damit die Bohrungen des Schienbeinknochens mit dem Fußspurbereich der Austrittsbohrungen am gegenüberliegenden Oberschenkelknochen zusammenpassen, wird der Bogenführungsrahmen 60 fixiert.

In einem ersten Arbeitsgang wird im Arbeitsfutterrohr 52 eine zentrische Führungsbohrung mit kleinem Durchmesser und beispielsweise mit einer Kirschner-Nadel hergestellt. Diese kleine Bohrung dringt durch die Kortikalis der Knochenseitenwandung in den Knochen ein und durchbohrt die Sponiosa, jedoch ohne die gegenüberliegende Kortikalis im Bereich der Gelenkkontaktfläche zu durchbohren.

Nun wird eine größere Ansatzbohrung 11 mit einem größeren Durchmesser 5 bis in die Spongiosa hergestellt. Dazu erfolgt eine Führung entlang der dünnen Führungsnadel, und auch diese große Bohrung durchdringt nicht die Kortikalis im Bereich der Kontaktfläche des Schienbeinknochens.

Der Durchmesser der Bohrung 11 liegt bevorzugterweise in einem Bereich zwischen 8 und 10 mm. Die vorausgegangene Zentrierbohrung mit der Kirschner-Nadel hat einem Durchmesser zwischen 2 und 3 mm.

Nun wird die Spongiosa aus der Bohrung 11 entfernt.

In das Arbeitsfutterrohr 52 wird nun ein Führungselement 54 mit schrägen Bohrführungen 56 eingebracht, über die Bohrungen mit kleinem Durchmesser 7 hergestellt werden können, die die Kontaktfläche in der Kortikalis durchbohren. Der Durchmesser dieser kleinen Schrägbohrungen 7 liegt bevorzugterweise in einem Bereich zwischen 2 und 4 mm.

Im Bereich des Schienbeins werden die Bohrungsöffnungen 40, 41 und 42, 43 bevorzugterweise zu Langlöchern 45, 46 vereint. Eine bevorzugte Ausführung dieser Langlöcher 45, 46 trägt die Abmessungen von 7 x 3,5 mm.

Die Langlöcher können auf unterschiedliche Weise erzeugt werden. Entweder wird durch Eindringen einer feilenartigen Vorrichtung das Langloch erzeugt oder durch Drehen des Führungselementes 54 um 180°.

Fig. 8 und Fig. 9 zeigen in einer 3-D-Zeichnung, wie die kleinen Bohrspitzen 66, 67 aus der Kortikalis austreten.

Die Herstellung der Bohrungen 40, 41, 42, 43 kann mit einzelnen Bohrern hergestellt werden oder durch zwei gleichzeitig eingedrehte Bohrer.

Wenn die einzelnen Bohrungen am Oberschenkelknochen und Schienbein hergestellt sind, werden die Ersatzkreuzbänder durch die einzelnen Bohrungen gezogen und im Schienbeinknochen in den zwei Langlöchern 45, 46 vereint. Die Endverankerung der Ersatzkreuzbänder in den seitlich an den Knochen liegenden Öffnungen erfolgt nach dem Stand der Technik.

## Patentansprüche

1. Verfahren zum Einbau von Ersatzkreuzbändern im Knie, **dadurch gekennzeichnet,**
- **dass** vom Seitenbereich des Oberschenkelknochens (1) und/oder des Seitenbereichs des Schienbeinknochens (2) eine Ansatzbohrung (10, 11) mit einem größeren Durchmesser (4, 5) eingebracht wird, die bevorzugter Weise in der Spongiosa endet und nicht durch die Kortikalis und Knochenhaut austritt und
- **dass** durch diese Ansatzbohrungen (10, 11) bezüglich deren Bohrachse mehrere Schrägbohrungen (6, 7) eingebaut werden, die einen kleineren Durchmesser haben, als die Ansatzbohrungen (10, 11) und
- **dass** diese kleineren Bohrungen (6, 7) so nach außen geneigt sind, dass ihre Austrittsöffnungen (20, 21, 22, 23, 40, 41, 42, 43) in der Kortikalis und Knochenhaut auf einer bevorzugt ovalen Fläche (24, 44) liegen, die größer ist als die Kreisquerschnittsfläche der Ansatzbohrungen (10, 11).

2. Verfahren nach Anspruch 1, **dadurch gekennzeichnet, dass** die Ansatzbohrungen (10, 11) einen Bohrdurchmesser (4, 5) in einem bevorzugten Bereich von 8 bis 12 mm haben und die Schrägbohrungen (6, 7) einen bevorzugten Bohrdurchmesser von 2 bis 5 mm besitzen.

3. Verfahren nach einem der Ansprüche 1 oder 2, **dadurch gekennzeichnet, dass** sich die Austrittsöffnungen (20, 21, 22, 23) in der Kortikalis in einem bevorzugten Bereich einer Ellipse (24) befinden, mit den Achsen (28) von etwa 14 bis 20 mm und den Achsen (29) von etwa 8 bis 12 mm.

4. Verfahren nach einem der Ansprüche 1 bis 3, **dadurch gekennzeichnet, dass** einzelne benachbarte Austrittsöffnungen (40, 41, 42, 43) zu Langlöchern (45, 46) mit glatten Rändern nachgearbeitet werden.

5. Verfahren nach einem der Ansprüche 1 bis 4, **dadurch gekennzeichnet, dass** Langlöcher (45, 46) im Schienbeinknochen und/oder im Oberschenkelknochen ausgeführt werden.

6. Verfahren nach einem der Ansprüche 1 bis 5, dadurch g e - **kennzeichnet**, dass das Verfahren mindestens die folgenden Arbeitsschritte enthält:
- zielgenaue Ausrichtung eines Führungsrahmens (50) mit einem Arbeitsfutterrohr (52) auf der einen Knochenseite und einem Festhalter (51) auf der gegenüberliegenden Kortikalis eines ersten Knochens
- Eindrücken eines Arbeitsfutterrohres (52) mit Zähnen (53) in die Kortikalis.
- Herstellen einer zentrischen Führungsbohrung im Arbeitsfutterrohr (52) mit kleinen Durchmesser, beispielsweise mit einer Kirschner-Nadel, durch die Spongiosa, ohne die gegenüberliegende Kortikalis im Bereich der Gelenk-Kontaktfläche zu durchbohren,
- Herstellung einer Bohrung mit größerem Durchmesser (4) in der Spongiosa, die die gegenüberliegende Kortikalis nicht durchdringt und die gegebenenfalls durch die Kirschner-Nadel geführt ist,
- Entfernung der Spongiosa aus der Bohrung (10) und Einführung eines länglichen Führungselementes (54) in das Arbeitsfutterrohr (52), durch das gegenüber der Bohrachse (58) der Bohrung (10) unter Winkeln (57) mehrere nach außen gerichtete Bohrungen (6) mit Bohrachsen (12) und mit kleinerem Durchmesser ausgeführt werden,
- und dass die gegenüberliegende Kortikalis mit mehreren unterschiedlich geneigten Bohrungen (6) kleineren Durchmessers durchbohrt wird, die mehrere beabstandete Austrittsöffnungen (20, 21, 22, 23) in der Kortikalis herstellen.

7. Verfahren nach einem der Ansprüche 1 bis 5, **dadurch gekennzeichnet, dass** es mindestens folgende Arbeitsschritte enthält:
- zielgenaue Ausrichtung eines Bogenführungsrahmens (60) mit einem Arbeitsfutterrohr (52) und mit Sägezähnen auf der Knochenseitenfläche und einem Spitzzahnhaken (64) auf der gegenüberliegenden Kortikalis eines weiteren Knochens , wobei die Ausrichtung auf den Fußspurbereich der Austrittsöffnungen (20, 21, 22, 23) der Bohrungen (6) am gegenüberliegenden Knieknochen erfolgt.
- Herstellen einer zentrischen Führungsbohrung im Arbeitsfutterrohr (52) mit kleinen Durchmesser, beispielsweise mit einer Kirschner-Nadel, durch die Spongiosa, ohne die gegenüberliegende Kortikalis im Bereich der Gelenk-Kontaktfläche zu durchbohren,
- Herstellung einer Bohrung (11) mit größerem Durchmesser und zentriert über die dünne Führungsnadel, ohne die Kortikalis zu durchdringen,
- Entfernung der Spongiosa aus der Bohrung (11),
- Einbau eines Führungselementes (54) in das Arbeitsfutterrohr (52) für nach außen geneigte kleinere Schrägbohrungen (7), die gegenüber der Achse der Bohrung (11) bzw. des Arbeitsfutterrohres (52) unter Winkeln (57) geneigt sind,
- Herstellung mehrerer nach außen geneigter, kleiner Schrägbohrungen (7) durch die Kortikalis, die im Bereich der Austrittsfläche zu Langlöchern (45, 46) nachgearbeitet werden.

8. Vorrichtung für das Verfahren nach Anspruch 1 bis 7, dadurch g e - **kennzeichnet**, dass sich an einem schraubzwingenartigen Führungsrahmen (50, 60) ein Arbeitsfutterrohr (52) mit Sägezähnen (53) befindet und dass auf der gegenüberliegenden Seite ein Festhalter (51) und/oder mindestens ein Spitzzahnhaken (64) angeordnet ist, die auf die Oberfläche der Knochen angepasst sind.

9. Vorrichtung nach Anspruch 8, **dadurch gekennzeichnet, dass** der Festhalter (51) und/oder der Spitzzahnhaken (64) so ausgestaltet sind, dass sie Öffnungen (55) oder Aussparungen aufweisen, durch die die Bohrköpfe der für die kleineren Bohrungen (6, 7, 20, 21, 22, 23, 40, 41, 42, 43) verwendeten Bohrer oder Kirschner-Nadeln (61, 66, 67, 58) frei aus der Kortikalis austreten können.

10. Vorrichtung nach einem der Ansprüche 8 oder 9, dadurch g e - **kennzeichnet**, dass in das Arbeitsfutterrohr (52) längliche Führungselemente (54) für kleinere Bohrungen (6, 7) eingeführt werden, die eine oder mehrere geneigte Bohrungen und/oder Führungsrillen (56) aufweisen, deren Richtung (59) bezüglich der Achse (58) des Arbeitsfutterrohres (52) einen Winkel (57) aufweisen.

## Geänderte Patentansprüche

### Geänderte Patentansprüche gemäss Regel 137(2) EPÜ.

1. Vorrichtung für ein Verfahren zum Einbau von Ersatzkreuzbändern im Knie,
gemäß dem
- vom Seitenbereich des Oberschenkelknochens (1) und/oder des Seitenbereichs des Schienbeinknochens (2) eine Ansatzbohrung (10, 11) mit einem größeren Durchmesser (4, 5) eingebracht wird, die bevorzugter Weise in der Spongiosa endet und nicht durch die Kortikalis und Knochenhaut austritt und
- durch diese Ansatzbohrungen (10, 11) bezüglich deren Bohrachse mehrere Schrägbohrungen (6, 7) eingebaut werden, die einen kleineren Durchmesser haben, als die Ansatzbohrungen (10, 11) und
- diese kleineren Bohrungen (6, 7) so nach außen geneigt sind, dass ihre Austrittsöffnungen (20, 21, 22, 23, 40, 41, 42, 43) in der Kortikalis und Knochenhaut auf einer bevorzugt ovalen Fläche (24, 44) liegen, die größer ist als die Kreisquerschnittsfläche der Ansatzbohrungen (10, 11),
wobei sich an einem schraubzwingenartigen Führungsrahmen (50, 60) der Vorrichtung ein Arbeitsfutterrohr (52) mit Sägezähnen (53) befindet und auf der gegenüberliegenden Seite ein Festhalter (51) und/oder mindestens ein Spitzzahnhaken (64) angeordnet ist/sind, die auf die Oberfläche der Knochen angepasst sind,
**dadurch gekennzeichnet**dass,
die Vorrichtung längliche Führungselemente (54) für die kleineren Bohrungen (6, 7) umfasst, die in das Arbeitsfutterrohr (52)eingeführt werden können, wobei die längliche Führungselemente (54) eine oder mehrere geneigte Bohrungen und/oder Führungsrillen (56) aufweisen, deren Richtung (59) bezüglich der Achse (58) des Arbeitsfutterrohres (52) einen Winkel (57) aufweisen.

2. Vorrichtung nach Anspruch 1, **dadurch gekennzeichnet dass**, der Festhalter (51) und/oder der Spitzzahnhaken (64) so ausgestaltet sind, dass sie Öffnungen (55) oder Aussparungen aufweisen, durch die die Bohrköpfe der für die kleineren Bohrungen (6, 7, 20, 21, 22, 23, 40, 41, 42, 43) verwendeten Bohrer oder Kirschner-Nadeln ( 61, 66, 67, 58 ) frei aus der Kortikalis austreten können.
